Europäisches Patentamt

European Patent Office

Office européen des brevets

(19)

(11) Publication number: **0 236 023**
**A2**

(12) **EUROPEAN PATENT APPLICATION**

(21) Application number: 87301472.4

(22) Date of filing: 20.02.87

(51) Int. Cl.⁴: **G 01 N 33/66**
C 12 Q 1/54
// G01N33/52

(30) Priority: 03.03.86 US 835800

(43) Date of publication of application:
09.09.87 Bulletin 87/37

(84) Designated Contracting States: **DE FR GB IT**

(71) Applicant: **Blackman, Seymour Norman**
**1530 Palisade Avenue**
**Fort Lee New Jersey 07024 (US)**

(72) Inventor: **Blackman, Seymour Norman**
**1530 Palisade Avenue**
**Fort Lee New Jersey 07024 (US)**

(74) Representative: **Coleman, Stanley et al**
**MATHYS & SQUIRE 10 Fleet Street**
**London EC4Y 1AY (GB)**

(54) Non-invasive blood glucose level monitoring method.

(57) A non-invasive, real-time, user-performed, blood glucose level monitoring method determines the blood glucose level as a function of the glucose level of lachrymal fluid. A kit of parts for enabling performance of the monitoring method is also described.

EP 0 236 023 A2

Bundesdruckerei Berlin

**Description**

## NON-INVASIVE BLOOD GLUCOSE LEVEL MONITORING METHOD

### BACKGROUND OF THE INVENTION

#### 1. Field of the Invention

This invention generally relates to a blood glucose level monitoring method and, more particularly, to a non-invasive, real-time, user-performed method which determines the blood glucose level as a function of the glucose level of lachrymal fluids.

#### 2. Description of the Prior Art

The self-monitoring of one's blood glucose level is an important part of the diagnosis and treatment of such diseases as diabetes. Indeed, the blood glucose level is often used to determine the self-administered insulin dose. Hence, the blood glucose level is typically determined by the diabetic at least once every day.

The most accurate method of determining one's blood glucose level is to have one's blood drawn and analyzed either in a doctor's office or in a laboratory. However, one cannot daily run to a doctor's office or a laboratory for this purpose and, hence, one must have the capability to determine his or her own blood glucose level in the privacy of one's home and, preferably, in a simple, efficient and accurate manner.

One of the most prevalent techniques is for one to prick his or her own finger or other body part to draw blood, and then to deposit one or more such drops of blood onto a reagent carrier strip having a glucose testing substance thereon. One common type of reagent strip is sold by Bio-Dynamics, Inc. under the trademark CHEMSTRIPS. The testing substances change color or shading in correspondence with the detected amount of blood glucose. Hence, one can consult a color chart typically provided in or on the side of a container in which the reagent strips are contained, and match the changed color of the testing substance with one of the colors on the chart. Once a match is made, a numerical value corresponding to the blood glucose level of the matched color on the color chart can be read. This information is used to select the correct insulin dose, and to advise the diabetic of the progress of his treatment.

Although very widespread in use, the above-described invasive technique is not altogether satisfactory, because the pricking procedure is somewhat messy and painful, particularly if one has to daily repeat the same procedure. Also, some individuals are rather squeamish at the sight of blood, particularly when it is their own. Experience has shown that many individuals forego the messiness and pain involved in this invasive technique, thereby leading to over-dosing or under-dosing of insulin and potential disaster. Also, the pricking technique is generally accomplished with the aid of a needle which is supposed to be sterilized before use. Experience has shown that many individuals, either through inadvertence or neglect, fail to sterilize the needle, thereby leading to infection and, even in the case of a properly sterilized needle, a wound is created which may become infected.

A less painful, non-invasive technique has been proposed in the prior art, and involves the testing of one's urine with appropriate reagent strips. Although generally satisfactory for its intended purpose, the known urine testing techniques detect blood glucose levels which are not representative of the current blood glucose level, but, rather, an average blood glucose level of those which existed several hours previously. Even so, such urine testing techniques have not proven to be altogether accurate in the testing of low blood glucose levels. Hence, the urine testing techniques do not provide instantaneous, i.e. real time, data, nor are they altogether reliable.

### SUMMARY OF THE INVENTION

#### 1. Objects of the Invention

It is a general object of this invention to overcome the aforementioned drawbacks of the prior art blood glucose level monitoring methods.

It is another object of this invention to provide a highly accurate, non-invasive, real-time, user-performed blood glucose level monitoring method.

It is a further object of this invention to provide such a method which is easy and inexpensive to perform without inflicting pain, without being unduly messy, and without promoting infection.

#### 2. Features of the Invention

In keeping with these objects, and others which will become apparent hereinafter, one feature of this invention resides, briefly stated, in a non-invasive, real-time, user-performed blood glucose level monitoring method which comprises the following steps:

First, lachrymal fluid glucose level is determined from lachrymal fluid obtained from lacrymal glands. For this purpose, it is desirable to provide a carrier having a blood glucose testing substance thereon, and to apply to the carrier said lachrymal fluid so that the testing substance reacts therewith. The reaction of the lachrymal fluid with the testing substance causes the color of the latter to change, thereby indicating the glucose level of the lachrymal fluid.

Thereupon, the blood glucose level is determined as a function of the lachrymal fluid glucose level. For this purpose, a chart, preferably a color chart, is provided with a plurality of colors alongside each of which numerals indicative of the blood glucose level are provided. In use, one visually compares the changed color of the testing substance with a corresponding color on the color chart and, once a match has been made, it is merely necessary for one to read the corresponding numeral adjacent the matched color on the chart to determine the blood glucose level.

Hence, it will be seen that this invention proposes a non-invasive technique which determines one's blood glucose level on a real-time basis. This method is easy to perform, without inflicting pain, without being unduly messy, and without promoting infection.

In one embodiment of the invention the lachrymal fluid is obtained by stimulating the flow thereof from the lachrymal glands suitably by wetting said glands with a non-isotonic saline solution which may be supplied to the glands by an eye dropper. The carrier with the testing substance thereon is then positioned over the glands to receive the lachrymal fluid which effects the discoloration of the testing substance.

The invention further comprises a kit for determining blood glucose level comprising a carrier bearing a substance which changes color when lachrymal fluid is applied thereto to an extent dependent upon the glucose level in said lachrymal fluid and a calibrated color chart with numerals indicative of blood glucose level whereby visual comparison between a carrier bearing said substance and subjected to color change by lachrymal fluid and said color chart enables blood glucose level to be ascertained.

The novel features which are considered as characteristic of this invention are set forth in particular in the appended claims. The invention itself, however, both as to its method of use, together with additional objects and additions thereof, best will be understood from the following description of specific embodiments.

## DETAILED DESCRIPTION OF THE PREFERRED EMBODIMENTS

In accordance with this invention, the blood glucose level is conveniently monitored by the patient himself in a non-invasive manner and on a real-time basis as a function of the glucose level of the lachrymal fluid issuing from the lachrymal glands, i.e. the tear ducts in the inner corners of one's eyes.

The blood glucose level is determined in the following manner:

First, the flow of lachrymal fluid from the lachrymal glands is stimulated, preferably by dropping one or more eye drops of a non-isotonic saline solution, desirably one of high saline concentration, into one's eyes.

Next, the lachrymal fluid glucose level is determined by positioning a carrier, preferably, a reagent strip having a blood glucose testing substance thereon, over the lachrymal glands so that the testing substance reacts with the flowing lachrymal fluid, thereby changing the color of the testing substance. Advantageously, the reagent strips may be those currently marketed by Bio-Dynamics, Inc. under the trademark CHEMSTRIPS.

Next, the blood glucose level is determined as a function of the lachrymal fluid glucose level. For this purpose, a chart, preferably a color chart, is provided in or on the exterior of a container in which the reagent strips are contained. The color chart is provided with a set of colors adjacent each of which are provided numerals indicative of various blood glucose levels and, more particularly, of blood glucose ranges. These numerals are calibrated such that they indicate the blood glucose level corresponding to the various colors on the chart. It is merely necessary for one to visually compare the changed color of the testing substance with the corresponding color on the color chart to determine the blood glucose level.

The above-described method does not involve the pricking of one's finger or other body part and, hence, is extremely easy to perform, even by the most squeamish and most clumsy individual. Wound infections are reliably prevented. This invention represents a significant achievement for diabetics to assist them in monitoring their blood glucose levels, because they can perform the above-described method as frequently as desired without the need for cumbersome and expensive medical equipment. This invention recognizes that the glucose level in lachrymal fluids is a function of the glucose level in blood.

It will be understood that each of the steps described above, or two or more together, also may find a useful application in other fields differing from the diabetic testing application described above.

Without further analysis, the foregoing will so fully reveal the gist of the present invention that others can, by applying current knowledge, readily adapt it for various applications without omitting features that, from the standpoint of prior art, fairly constitute essential characteristics of the generic or specific aspects of this invention and, therefore, such adaptations should and are intended to be comprehended within the meaning and range of equivalence of the following claims.

**Claims**

1. A non-invasive, real-time, blood glucose level monitoring method, comprising the steps of:

(a) stimulating the flow of lachrymal fluid from the lachrymal glands;

(b) determining the lachrymal fluid glucose level from the lachrymal fluid; and

(c) determining the blood glucose level as a function of the lachrymal fluid glucose level.

2. The method as recited in claim 1, wherein the stimulating step includes the step of wetting the lachrymal glands with a non-isotonic saline solution.

3. The method as recited in claim 1, wherein the step (b) includes the step of occluding the lachrymal glands and collecting the lacrymal fluids flowing therefrom.

4. The method as recited in claim 3, wherein the step (b) includes the steps of providing a carrier having a blood glucose testing substance thereon, covering the lachrymal glands with the carrier, and collecting the lachrymal fluids on the carrier for reaction with the testing substance.

5. The method as recited in claim 1, wherein the step (c) includes the step of calibrating a chart with numerals indicative of the blood glucose level as a function of the lachrymal fluid glucose level.

6. The method as recited in claim 5, wherein the step (b) includes the step of reacting a blood glucose testing substance with the flowing lachrymal fluid to change the color of the substance, and wherein the chart is a color chart having a plurality of different colors corresponding to different blood glucose levels, and wherein the step (c) includes the step of visually comparing the changed color of the testing substance with a corresponding color on the color chart.

7. The method as recited in claim 1, wherein the steps (a), (b) and (c) are all performed by a user whose blood glucose level is to be monitored.

8. A non-invasive, real-time, user-performed, blood glucose level monitoring method as a function of lachrymal fluid glucose level, comprising the steps of:

(a) stimulating the flow of lachrymal fluid from the lachrymal glands by wetting the same with a non-isotonic saline solution;

(b) providing a blood glucose testing substance on a carrier;

(c) positioning the carrier over the lachrymal glands so that the testing substance reacts with the flowing lachrymal fluids, thereby changing the color of the testing substance and indicating the lachrymal fluid glucose level; and

(d) determining the blood glucose level as a function of the lachrymal fluid glucose level by calibrating a color chart with numerals indicative of the blood glucose level, and visually comparing the changed color of the testing substance with a corresponding color on the color chart.

9. A non-invasive, real-time, blood glucose level monitoring method characterised by:

(a) determining the lachrymal fluid glucose level from lachrymal fluid obtained from a human or other animal; and

(b) determining the blood glucose level as a function of said determined lachrymal fluid glucose level.

10. The method claimed in Claim 9, characterised by providing a blood glucose level testing substance on a carrier, applying said lachrymal fluid to said substance on said carrier thereby changing the color of said substance to indicate the lachrymal fluid glucose level and determining the blood glucose level as a function of the lachrymal fluid glucose level by calibrating a color chart with numerals indicative of the blood glucose level, and visually comparing the changed color of the testing substance with a corresponding color on the color chart.

11. A kit for determining blood glucose level comprising a carrier bearing a substance which changes color when lachrymal fluid is applied thereto to an extent dependent upon the glucose level in said lachrymal fluid and a calibrated color chart with numerals indicative of blood glucose level whereby visual comparison between a carrier bearing said substance and subjected to color change by lachrymal fluid and said color chart enables blood glucose level to be ascertained.